# EUROPÄISCHE PATENTSCHRIFT

(11) **EP 2 560 666 B1**
(45) Veröffentlichungstag und Bekanntmachung des Hinweises auf die Patenterteilung: **24.10.2018**
(21) Anmeldenummer: 11709434.2
(22) Anmeldetag: 23.03.2011
(51) Int. Cl.: A61K 31/7012, A61K 31/7028, A61K 36/575, A61K 8/06, A61K 8/34, A61K 8/37, A61K 8/39, A61K 8/60, A61K 8/97, A61Q 19/00

(54) **WIRKSTOFFKOMBINATIONEN AUS MAGNOLIENRINDENEXTRAKT UND OBERFLÄCHENAKTIVEN AGENTIEN (POLYGLACERYL(3)-METHYLGLUCOSEDISTEARAT)**
AGENT COMBINATIONEN OF MAGNOLIA BARK EXTRACT AND SURFACTACE ACTIVE AGENTS (POLYGLACERYL(3)-METHYLGLUCOSEDISTEARATE)
COMBINAISONS DES AGENTS AVEC UN EXTRAIT D' ECORCE DE MAGNOLIA ET DES AGENTS ACTIFS TENSIOACTIFS (POLYGLACERYL(3)-METHYLGLUCOSEDISTEARATE)

(30) Priorität: 20.04.2010 DE 102010015792
(43) Veröffentlichungstag der Anmeldung: 27.02.2013
(73) Patentinhaber: Beiersdorf AG, 20253 Hamburg (DE)
(72) Erfinder: SCHLÄGER, Torsten, 22297 Hamburg (DE); ECKERT, Julia, 20249 Hamburg (DE); NEUFANG, Gitta, 20149 Hamburg (DE); PETERS, Nils, 22965 Todendorf (DE); KNAUPMEIER, Stefanie, 32108 Bad Salzuflen (DE); HEUSER, Stefan, 90762 Fürth (DE)
(86) Internationale Anmeldenummer: PCT/EP2011/054401
(87) Internationale Veröffentlichungsnummer: WO 2011/131441

(56) Entgegenhaltungen:
- WO-A1-03/030858
- WO-A1-2007/064519
- DE-A1- 10 105 904
- DATABASE BIOSIS [Online] BIOSCIENCES INFORMATION SERVICE, PHILADELPHIA, PA, US; Juli 2001 (2001-07), CHEN YUH-LIEN ET AL: "Magnolol, a potent antioxidant from Magnolia officinalis, attenuates intimal thickening and MCP-1 expression after balloon injury of the aorta in cholesterol-fed rabbits", XP002633717, Database accession no. PREV200200119609 & BASIC RESEARCH IN CARDIOLOGY, Bd. 96, Nr. 4, Juli 2001 (2001-07), Seiten 353-363, ISSN: 0300-8428

## Beschreibung

Die vorliegende Erfindung betrifft kosmetische oder dermatologische Zubereitungen zur Behandlung, Pflege und Prophylaxe von sensibler Haut und erythematösen Erscheinungen.

Das Problem "sensible Haut" betrifft eine wachsende Anzahl Erwachsener und Kinder. Mit sensibler Haut bezeichnet man eine Kombination verschiedener Symptome, wie hyperreaktive und intolerante Haut. Aber auch atopische Haut kann darunter subsummiert werden. Diese Hautzustände werden von den Betroffenen oftmals, nicht ganz korrekt, als "allergische" Haut bezeichnet. Obwohl eine allergische Erkrankung zu Symptomen der sensiblen Haut führen kann, ist die Erscheinung "sensible Haut" nicht auf Allergiker beschränkt.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismus spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann ein mit "Stinging" (<engl.> "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

Bei atopischer Haut ist der Juckreiz als neurosensorisches Phänomen anzusehen.

"Stinging"-Phänomene können als kosmetisch zu behandelnde Störungen angesehen werden. Starker Juckreiz dagegen, insbesondere bei Atopie auftretendes starkes Hautjucken, kann auch als schwerwiegendere dermatologische Störung bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen z.B. Massage, Tensideinwirkung, Wettereinfluß wie Sonne, Kälte, Trockenheit, aber auch feuchte Wärme, Wärmestrahlung und UV-Strahlung, z.B. der Sonne, hervorgerufen werden.

Nach bisherigen Erkenntnissen tritt eine derartige Empfindlichkeit gegenüber ganz bestimmten Substanzen individuell unterschiedlich auf. Dies bedeutet, eine Person, die bei Kontakt mit einer Substanz "Stingingeffekte" erlebt, wird sie mit hoher Wahrscheinlichkeit bei jedem weiteren Kontakt wiederholt erleben. Der Kontakt mit anderen "Stingern" kann aber ebensogut ohne jede Reaktion verlaufen.

Viele mehr oder weniger empfindliche Personen haben auch bei Verwendung mancher desodorierenden oder antitranspirierend wirkenden Zubereitungen unter erythematösen Hauterscheinungen zu leiden.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Daher sollten insbesondere Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, ins-besondere Dermatosen, aber auch des Erscheinungsbildes des "Stingings" zur Verfügung gestellt werden, die solche erythematösen Hauterscheinungen lindern.

Erfindungsgemäß werden diese Aufgaben gelöst durch die Verwendung von Magnolienrindenextrakt zur Herstellung von kosmetischen oder dermatologischen Zubereitungen zur Behandlung, Pflege und Prophylaxe von sensibler Haut und/oder zur Behandlung und Prophylaxe von erythematösen Erscheinungen.

Magnolienrindenextrakt ist bekanntermaßen ein antimikrobiell wirksames Agens und wurde seit langem in der indianischen Volksmedizin verwendet. Nachteilig ist, daß solche Extrakte leichtverderblich sind, und sich bereits in kurzer Frist durch Lichteinfall, Luftzutritt und Temperatureinflüsse dunkel zu verfärben beginnen, wodurch ihre Wirksamkeit meßbar abnimmt.

Eine weitere Aufgabe der vorliegenden Erfindung bestand daher darin, Wirkstoffe bzw. Zubereitungen, solche Wirkstoffe enthaltend, zur Verfügung zu stellen, die sich durch eine erhöhte Beständigkeit gegenüber Licht- und/oder Lufteinwirkung und/oder Temperatureinflüssen auszeichnen würden.

Es wurde überraschend gefunden, und darin liegt die Lösung all dieser Aufgaben, daß Wirkstoffkombinationen aus
a) einem Magnolienrindenextrakt mit einem Gehalt an Magnolol und/oder Honokiol und
b) einer oder mehreren grenzflächenaktiven Substanzen A, gewählt aus der Gruppe der Glucosederivate, welche sich durch die Strukturformel 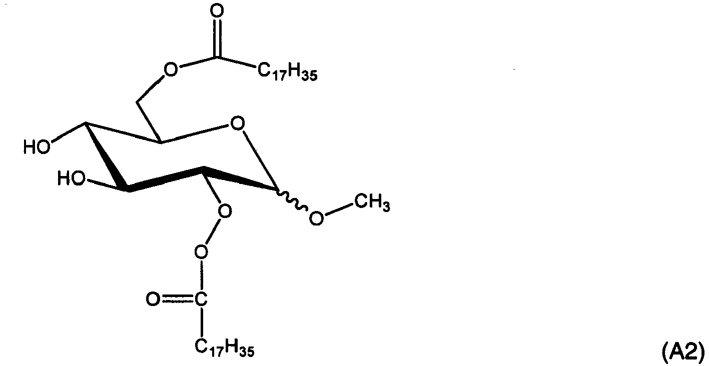 auszeichnen, und
(c) einer oder mehreren grenzflächenaktiven Substanzen B, gewählt aus der Gruppe der Substanzen der allgemeinen Strukturformel 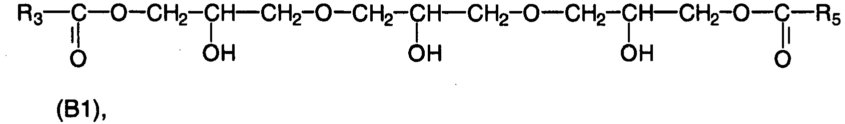 wobei R3 und R5 unabhängig voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt,
dadurch gekennzeichnet, dass ein ungefähr äquimolekulares Gemisch aus den Verbindungen A2 und B1, wobei in B1 die Reste R3 und R5 vorzugsweise beide einen Stearatrest bezeichnen, gewählt werden, bekannt als "Polyglyceryl(3)-Methylglucosedistearat, den Nachteilen des Standes der Technik abhelfen.

Schließlich besteht eine vorteilhafte Ausführungsform der vorliegenden Erfindung in der Verwendung
b) einer oder mehreren grenzflächenaktiven Substanzen A, gewählt aus der Gruppe der Glucosederivate, welche sich durch die Strukturformel 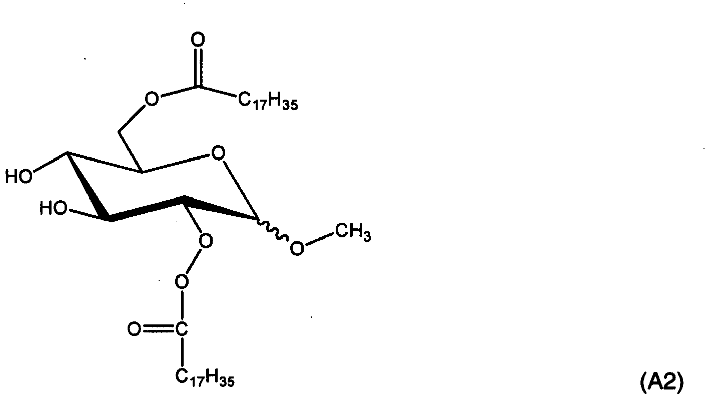 auszeichnen, und
(c) einer oder mehreren grenzflächenaktiven Substanzen B, gewählt aus der Gruppe der Substanzen der allgemeinen Strukturformel 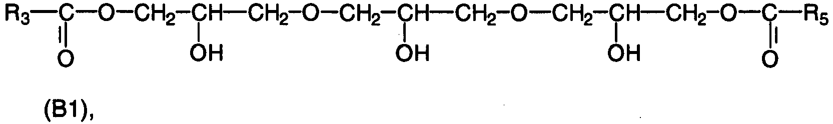 wobei R3 und R5 unabhängig voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt,
dadurch gekennzeichnet, dass ein ungefähr äquimolekulares Gemisch aus den Verbindungen A2 und B1, wobei in B1 die Reste R3 und R5 vorzugsweise beide einen Stearatrest bezeichnen, gewählt werden, bekannt als "Polyglyceryl(3)-Methylglucosedistearat"
zur Stabilisierung eines
c) Magnolienrindenextraktes mit einem Gehalt an Magnolol und/oder Honokiol gegen den Verderb durch Licht- und/oder Lufteinwirkung und/oder Temperatureinflüsse.

Bei Anwendung der erfindungsgemäßen Wirkstoffkombinationen ist eine wirksame Behandlung, aber auch eine Prophylaxe von entzündlichen Hautzuständen - auch dem atopischen Ekzem - möglich. Kosmetische oder topische dermatologische Zubereitungen mit einem wirksamen Gehalt an den erfindungsgemäßen Wirkstoffkombinationen dienen aber auch in überrachender Weise zur Beruhigung von empfindlicher oder gereizter Haut.

Magnolien (Magnolia) sind eine Pflanzengattung in der Familie der Magnoliengewächse (Magnoliaceae). Diese enthält etwa 230 Arten, die aus Ostasien und Amerika stammen. Erfindungsgemäß kann der Magnolienrindenextrakt grundsätzlich allen Arten der Gattung Magnolia entnommen werden, wobei allerdings Magnolia grandiflora, und insbesondere Magnolia officinalis bevorzugt werden.

Magnolien-Rinde wird in der Traditionellen Chinesischen Medizin (TCM) vor allem bei "Stagnation of qi" (low energy), emotionalem Stress, Entzündungen, innerer Unruhe und Angstzuständen eingesetzt.

Die Herstellung des verwendeten Extraktes erfolgt durch Extraktion mit Hilfe von superkritischem CO₂ oder Ethanol oder einer Ethanol / Wasser Mischung.
Als typische Inhaltstoffe und Leitsubstanzen Leitsubstanzen des Extraktes sind Magnolol und Honokiol definiert.

Magnolol ist durch folgende Struktur gekennzeichnet:

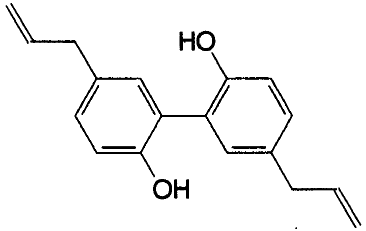

Honokiol ist durch folgende Struktur gekennzeichnet:

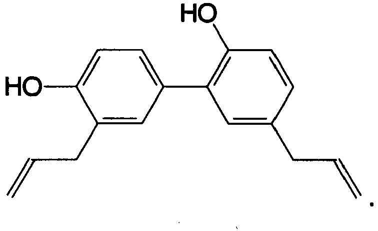

Erfindungsgemäß werden die Wirkstoffkombinationen bevorzugt in kosmetischen oder dermatologischen Zusammensetzungen eingesetzt mit einem Gehalt von 0,005 - 50,0 Gew.-%, insbesondere 0,01 - 20,0 Gew.-%, bezogen auf das Gesamtgewicht der Zusammensetzung. Vorteilhaft enthalten die Zusammensetzungen 0,02 - 10,0 Gew.-%, besonders bevorzugt 0,02 - 5,0 Gew.-% an den Wirkstoffkombinationen, ganz besonders vorteilhaft 0,1 - 3,0 Gew.-%, jeweils bezogen auf das Gesamtgewicht der Zusammensetzung.

Als erfindungsgemäß bevorzugte Emulgatorkombinationen sind als "Polyglyceryl(3)-Methylglucosedistearat" (PGMS) unter der Warenbezeichnung Tego Care® 450 von der Gesellschaft Evonik erhältlich.

Die erfindungsgemäß verwendeten Wirkstoffkombinationen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

Es ist auch gegebenenfalls vorteilhaft, die Zusammensetzungen gemäß der Erfindung abzupuffern. Vorteilhaft ist ein pH-Bereich von 3,5 - 7,5. Besonders günstig ist es, den pH-Wert in einem Bereich von 4,0 - 6,5 zu wählen.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetischen und dermatologischen Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-ÖI (W/O) oder vom Typ Öl-in-Wasser (O/W), oder eine multiple Emulsionen, beispielsweise vom Typ Wasser-in-ÖI-in-Wasser (W/O/W), ein Gel, eine Hydrodispersion, einen festen Stift oder auch ein Aerosol darstellen.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdikkungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, mindestens eine dialkylsubstituierte Carbonsäure im wäßrigen Medium und Hilfsmittel, wie sie üblicherweise dafür verwendet werden.

### Wirkungsnachweis

Magnolienrindenextrakt enthaltend Magnolol und / oder Honokiol weist bei Lagerung im Licht sowie bei höheren Temperaturen (Brutschrank 40°C) eine Instabilität auf. Diese Instabilität ist vermutlich auf eine Zersetzung der beiden aktiven Komponenten Magnolol und Honokiol zurückzuführen und äußert sich in einer Verfärbung des Materials.

Auch in kosmetischen Formulierungen kann diese Instabilität normalerweise nicht verhindert werden. Auch hier wird nach längerer Lichtexposition bzw. Lagerung bei erhöhter Temperatur (40°C) eine Verfärbung des Ansatzes beobachtet.

Durch die Verwendung eines erfindungsgemäß ausgewählten O/W-Emulgators (Polygylceryl-3-Methylglucosedistearat) konnte überraschenderweise eine Stabilisierung von Magnolienrindenextrakt in kosmetischen Formulierungen gezeigt werden. Sowohl nach Lichtexposition als auch nach Lagerung bei erhöhter Temperatur (40°C) kam es zu keiner Verfärbung der Ansätze.

### Beschreibung der Erfindung und der durchgeführten Versuche

Es wurden jeweils 3 Ansätze mit sechs verschiedenen Emulgatoren durchgeführt, bei denen jeweils der Emulgator alleine, der Magnolienrindenextrakt alleine und eine Mischung aus Emulgator und Magnolienrindenextrakt in Wasser und Butylenglykol gelöst wurden.

Als Emulgatoren wurden Polyglyceryl-3-Methylglucose Distearat, Na-Stearoyl Glutamat und Na-Cetearylsulfat (Mischung aus Na-Cetylsulfat und Na-Stearylsulfat) verwendet.

Die Einsatzmengen sowie die Versuchsbedingungen waren bei allen Ansätzen identisch (2g Emulgator, 0,5g Magnolienrindenextrakt, 10g Butylenglykol, Wasser ad 100g). Nach zwei Monaten Lagerung im Licht und bei erhöhter Temperatur (40°) wurde die Färbung der Ansätze durch ein Expert Grading mittels einer Farbschablone bewertet.

### Ergebnisse:

Die Auswertung mittels Farbschablone wurde folgendermaßen vorgenommen:
Anhand verschiedener Farbtafeln wurde zunächst die Farbe zu t₀ bestimmt. Die Farbveränderung nach t_{2 Monate} wird durch Δ x% angegeben.

Bewertung der Färbung der B40-Ansätze durch Expert Grading mittels Farbschablone

| | Magnolienrindenextrakt + Butylenglykol + H₂O | Emulgator + Magnolienrindenextrakt + Butylenglykol + H₂O |
|---|---|---|
| | t_{2Monate} | t_{2Monate} |
| Polyglyceryl-3-methylglucoseDistearat | Δ = 60% | Δ = 15% |
| Natrium-Stearoylglutamat | Δ = 70% | Δ = 80% |
| Natrium-Cetearylsulfat | Δ = 60% | Δ = 80% |

Bei Verwendung von Polyglyceryl-3-Methylglucosedistearat, konnte eine Stabilisierung von Magnolienrindenextrakt in kosmetischen Formulierungen gezeigt werden. Sowohl nach Lichtexposition als auch nach Lagerung bei erhöhter Temperatur (40 °C) kam es zu keiner Verfärbung der Ansätze.

Bei Verwendung der Emulgatoren Natriumcetearylsulfat und Natriumstearoylglutamat indessen war keine nennenswerte Stabilisierung zu beobachten.

Die folgenden Beispiele sollen die Verkörperungen der vorliegenden Erfindungen verdeutlichen. Die Angaben beziehen sich stets auf Gewichts-%, sofern nicht andere Angaben gemacht werden.

### Beispiel 1:

| | |
|---|---|
| Polyglyceryl-3 Methylglucose Distearat | 2,50 |
| Sorbitan Stearat | 1,50 |
| C12-15 Alkyl Benzoat | 2,50 |
| Caprylsäure/Caprinsäuretriglyceride | 2,50 |
| Stearylalkohol | 1,00 |
| Cyclomethicon | 3,00 |
| Dicaprylyl Carbonat | 2,50 |
| Glycerin | 3,00 |
| Methylparaben | 0,20 |
| Phenoxyethanol | 0,40 |
| Propylparaben | 0,10 |
| Carbomer | 0,10 |
| Magnolienrindenextrakt | 0,50 |
| Natriumhydroxid | q.s. |
| Parfüm | q.s. |
| Wasser | ad 100 |

### Beispiel 2:

| | |
|---|---|
| Polyglyceryl-3 Methylglucose Distearat | 2,50 |
| Sorbitanstearat | 3,00 |
| C12-15 Alkyl Benzoat | 2,50 |
| Caprylsäure/Caprinsäuretriglyceride | 2,50 |
| Stearylalkohol | 1,50 |
| Cyclomethicon | 1,00 |
| Dicaprylyl Carbonat | 2,50 |
| Dimethicon | 1,00 |
| Glycerin | 7,50 |
| Methylparaben | 0,20 |
| Phenoxyethanol | 0,40 |
| Propylparaben | 0,10 |
| Carbomer | 0,10 |
| Trinatrium EDTA | 1,00 |
| Tapioka Stärke | 1,00 |
| Distärke Phosphat | 1,00 |
| Magnolienrindenextrakt | 0,70 |
| Ethylhexylmethoxycinnamat | 3,00 |
| Butylmethoxydibenzoylmethan | 2,00 |
| Octocrylen | 1,00 |
| Natriumhydroxid | q.s. |
| Parfüm | q.s. |
| Wasser | ad 100 |

### Beispiel 3:

| | |
|---|---|
| Polyglyceryl-3-Methylglucose Distearat | 2,00 |
| Stearylalkohol | 3,00 |
| C₁₂₋₁₅ Alkylbenzoat | 3,00 |
| Butylenglycol Dicaprylat/Dicaprat | 2,00 |
| Caprylsäure/Caprinsäuretriglyceride | 3,00 |
| Dimethylpolysiloxan (Dimethicon) | 1,00 |
| Phenylbenzimidazolsulfonsäure | 1,00 |
| Bisoctyltriazol | 5,00 |
| Magnolienrindenextrakt | 0,30 |
| Phenoxyethanol | 0,40 |
| p-Hydroxybenzoesäurealkylester (Paraben) | 0,20 |
| Xanthan Gum | 0,10 |
| Carbomer | 0,20 |
| Butylenglycol | 5,00 |
| Additive (BHT, Puderrohstoffe, Farbstoffe) | 0,50 |
| Parfüm | q.s. |
| Wasser | ad 100 |

### Beispiel 4:

| | |
|---|---|
| Polyglyceryl-3 Methylglucosedistearat | 3,00 |
| Glyceryl Stearat | 1,00 |
| Stearylalkohol | 1,00 |
| Caprylsäure/Caprinsäuretriglyceride | 3,00 |
| Myristylmyristat | 1,00 |
| Cyclomethicon | 3,00 |
| Dicaprylylcarbonat | 4,00 |
| Sonnenblumenöl | 0,50 |
| Glycerin | 5,00 |
| Methylparaben | 0,30 |
| Phenoxyethanol | 0,40 |
| Carbomer | 0,10 |
| Trinatrium EDTA | 1,00 |
| Grüner Tee Extrakt | 0,05 |
| Niacinamid | 0,10 |
| Ubichinon | 0,10 |
| Tocopherylacetat | 0,20 |
| Panthenol | 0,50 |
| Magnolienrindenextrakt | 0,10 |
| Kamillenextrakt | 0,10 |
| Hammamelisextrakt | 0,10 |
| Carrageenan | 0,30 |
| Xanthan Gum | 0,10 |
| Natriumhydroxid | q.s. |
| Parfüm | q.s. |
| Wasser | ad 100 |

### Beispiel 3:

| | |
|---|---|
| Polyglyceryl-3-Methylglucose Distearat | 2,00 |
| Stearylalkohol | 3,00 |
| C₁₂₋₁₅ Alkylbenzoat | 3,00 |
| Butylenglycol Dicaprylat/Dicaprat | 2,00 |
| Caprylsäure/Caprinsäuretriglyceride | 3,00 |
| Dimethylpolysiloxan (Dimethicon) | 1,00 |
| Phenylbenzimidazolsulfonsäure | 1,00 |
| Bisoctyltriazol | 5,00 |
| Magnolienrindenextrakt | 0,30 |
| Phenoxyethanol | 0,40 |
| p-Hydroxybenzoesäurealkylester (Paraben) | 0,20 |
| Xanthan Gum | 0,10 |
| Carbomer | 0,20 |
| Butylenglycol | 5,00 |
| Additive (BHT, Puderrohstoffe, Farbstoffe) | 0,50 |
| Parfüm | q.s. |
| Wasser | ad 100 |

### Beispiel 4:

| | |
|---|---|
| Polyglyceryl-3 Methylglucosedistearat | 3,00 |
| Glyceryl Stearat | 1,00 |
| Stearylalkohol | 1,00 |
| Caprylsäure/Caprinsäuretriglyceride | 3,00 |
| Myristylmyristat | 1,00 |
| Cyclomethicon | 3,00 |
| Dicaprylylcarbonat | 4,00 |
| Sonnenblumenöl | 0,50 |
| Glycerin | 5,00 |
| Methylparaben | 0,30 |
| Phenoxyethanol | 0,40 |
| Carbomer | 0,10 |
| Trinatrium EDTA | 1,00 |
| Grüner Tee Extrakt | 0,05 |
| Niacinamid | 0,10 |
| Ubichinon | 0,10 |
| Tocopherylacetat | 0,20 |
| Panthenol | 0,50 |
| Magnolienrindenextrakt | 0,10 |
| Kamillenextrakt | 0,10 |
| Hammamelisextrakt | 0,10 |
| Carrageenan | 0,30 |
| Xanthan Gum | 0,10 |
| Natriumhydroxid | q.s. |
| Parfüm | q.s. |
| Wasser | ad 100 |

## Patentansprüche

1. Wirkstoffkombinationen aus
a) einem Magnolienrindenextrakt mit einem Gehalt an Magnolol und/oder Honokiol und
b) einer oder mehreren grenzflächenaktiven Substanzen A, gewählt aus der Gruppe der Glucosederivate, welche sich durch die Strukturformel 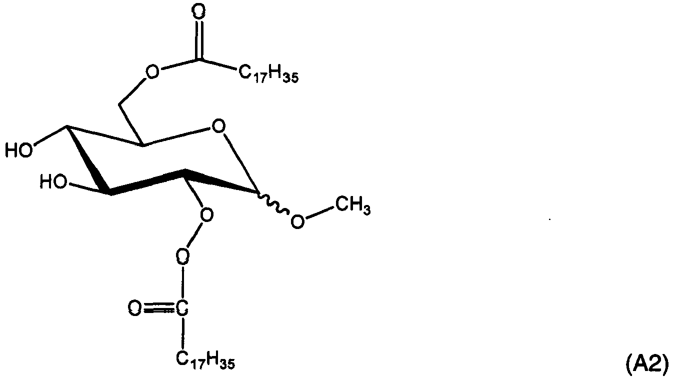 auszeichnen, und
(c) einer oder mehreren grenzflächenaktiven Substanzen B, gewählt aus der Gruppe der Substanzen der allgemeinen Strukturformel 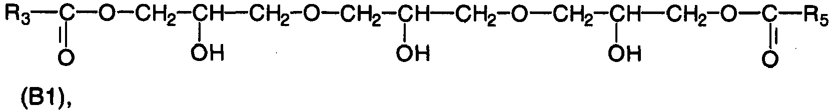 **dadurch gekennzeichnet, dass** ein ungefähr äquimolekulares Gemisch aus den Verbindungen A2 und B1, wobei in B1 die Reste R3 und R5 vorzugsweise beide einen Stearatrest bezeichnen, gewählt werden, bekannt als "Polyglyceryl(3)-Methylglucosedistearat".

2. Verwendung
b) einer oder mehreren grenzflächenaktiven Substanzen A, gewählt aus der Gruppe der Glucosederivate, welche sich durch die Strukturformel 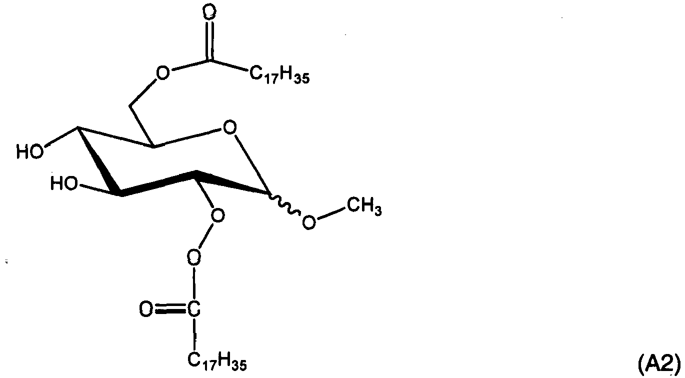 auszeichnen, und
(c) einer oder mehreren grenzflächenaktiven Substanzen B, gewählt aus der Gruppe der Substanzen der allgemeinen Strukturformel 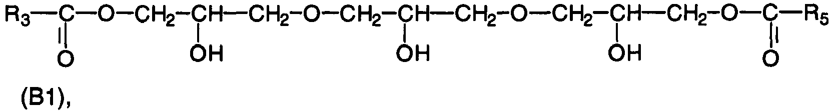 zur Stabilisierung eines
a) Magnolienrindenextraktes mit einem Gehalt an Magnolol und/oder Honokiol gegen den Verderb durch Licht- und/oder Lufteinwirkung und/oder Temperatureinflüsse, **dadurch gekennzeichnet, dass** ein ungefähr äquimolekulares Gemisch aus den Verbindungen A2 und B1, wobei in B1 die Reste R3 und R5 vorzugsweise beide einen Stearatrest bezeichnen, gewählt werden, bekannt als "Polyglyceryl(3)-Methylglucosedistearat".

3. Zubereitungen enthaltend Wirkstoffkombinationen gemäß Anspruch 1 mit einem Gehalt von 0,005 - 50,0 Gew.-%, insbesondere 0,01 - 20,0 Gew.-%, bevorzugt 0,02 - 10,0 Gew.- %, besonders bevorzugt 0,02 - 5,0 Gew, ganz besonders vorteilhaft 0,1 - 3,0 Gew.-%, jeweils bezogen auf das Gesamtgewicht der Zusammensetzung.

4. Verwendung nach Anspruch 2, bei der Wirkstoffkombinationen in kosmetischen oder dermatologischen Zubereitungen vorliegen, mit einem Gehalt von 0,005 - 50,0 Gew.-%, insbesondere 0,01 - 20,0 Gew.-%, bevorzugt 0,02 - 10,0 Gew.-%, besonders bevorzugt 0,02 - 5,0 Gew, ganz besonders vorteilhaft 0,1 - 3,0 Gew.-%, jeweils bezogen auf das Gesamtgewicht der Zusammensetzung.

5. Zubereitungen nach einem der vorstehenden Ansprüche, wobei als Magnolienrindenextrakt ein Extrakt aus der Rinde von Magnolia officinalis gewählt wird.

6. Verwendung nach einem der vorstehenden Ansprüche, wobei als Magnolienrindenextrakt ein Extrakt aus der Rinde von Magnolia officinalis gewählt wird.

## Claims

1. Active ingredient combinations of
a) a magnolia bark extract having a content of magnolol and/or honokiol and
b) one or more surface-active substances A, selected from the group of glucose derivatives, which are **characterized by** the structural formula 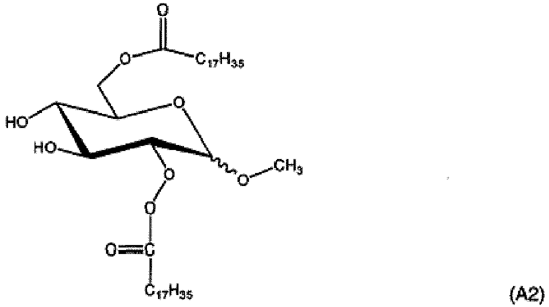 and
c) one or more surface-active substances B, selected from the group of substances of general structural formula 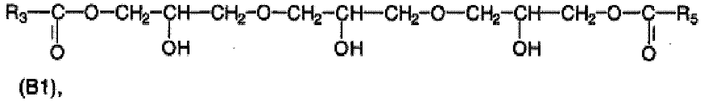 **characterized in that** an approximately equimolecular mixture of compounds A2 and B1 are selected, wherein in B1 the radicals R3 and R5 preferably both signify a stearate radical, known as "polyglyceryl-3 methylglucose distearate".

2. Use
b) of one or more surface-active substances A, selected from the group of glucose derivatives, which are **characterized by** the structural formula 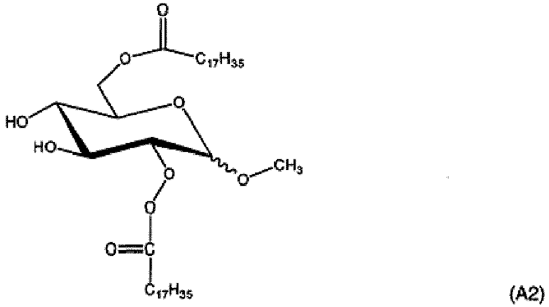 and
c) one or more surface-active substances B, selected from the group of substances of general structural formula 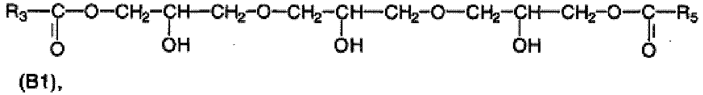 for stabilizing a
a) magnolia bark extract having a content of magnolol and/or honokiol against deterioration by the effect of light and/or air and/or the influence of temperature, **characterized in that** an approximately equimolecular mixture of compounds A2 and B1 are selected, wherein in B1 the radicals R3 and R5 preferably both signify a stearate radical, known as "polyglyceryl-3 methylglucose distearate".

3. Preparations comprising active ingredient combinations according to Claim 1 having a content of 0.005 - 50.0% by weight, particularly 0.01 - 20.0% by weight, preferably 0.02 - 10.0% by weight, particularly preferably 0.02 - 5.0% by weight, especially advantageously 0.1 - 3.0% by weight, based in each caseon the total weight of the composition.

4. Use according to Claim 2, in which active ingredient combinations are present in cosmetic or dermatological preparations, having a content of 0.005 - 50.0% by weight, particularly 0.01 - 20.0% by weight, preferably 0.02 - 10.0% by weight, particularly preferably 0.02 - 5.0% by weight, especially advantageously 0.1 - 3.0% by weight, based in each case on the total weight of the composition.

5. Preparations according to any of the preceding claims, wherein the magnolia bark extract selected is an extract of the bark of Magnolia officinalis.

6. Use according to any of the preceding claims, wherein the magnolia bark extract selected is an extract of the bark of Magnolia officinalis.

## Revendications

1. Combinaisons d'agents actifs constituées par :
a) un extrait d'écorce de magnolia ayant une teneur en magnolol et/ou honokiol, et
b) une ou plusieurs substances tensioactives A, choisies dans le groupe des dérivés de glucose, qui sont **caractérisées par** la formule structurale 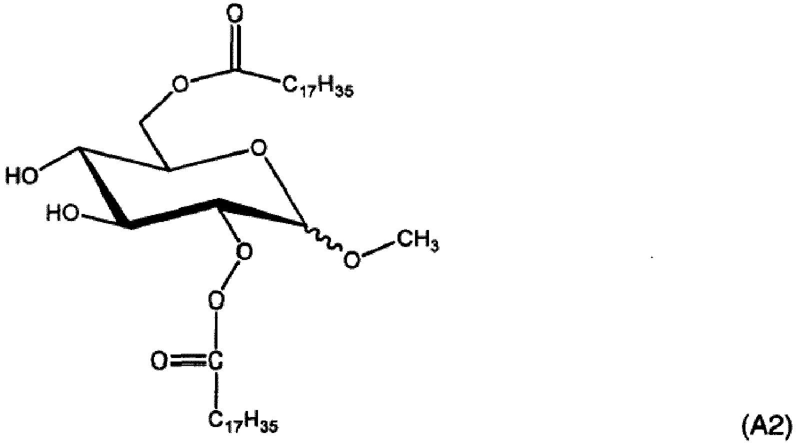 et
(c) une ou plusieurs substances tensioactives B, choisies dans le groupe des substances de formule structurale générale 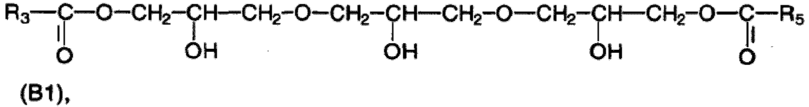 **caractérisées en ce qu'**un mélange approximativement équimolaire des composés A2 et B1, les radicaux R3 et R5 dans B1 désignant de préférence tous les deux un radical stéarate, est choisi, connu sous le nom de « distéarate de polyglycéryl(3)-méthylglucose ».

2. Utilisation de
b) une ou plusieurs substances tensioactives A, choisies dans le groupe des dérivés de glucose, qui sont **caractérisées par** la formule structurale 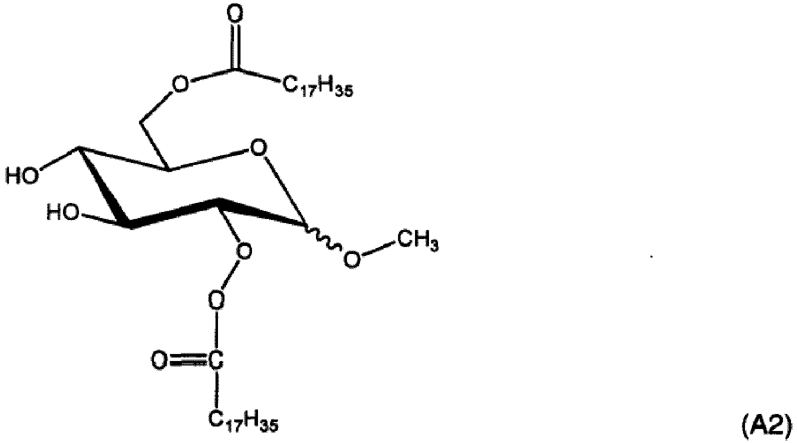 et
(c) une ou plusieurs substances tensioactives B, choisies dans le groupe des substances de formule structurale générale 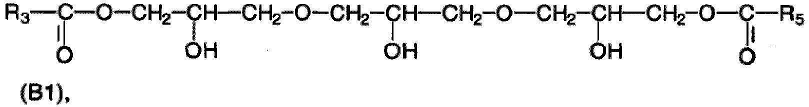 pour la stabilisation de
a) un extrait d'écorce de magnolia ayant une teneur en magnolol et/ou honokiol
contre la détérioration sous l'effet de la lumière et/ou de l'air et/ou sous l'effet de la température,
**caractérisée en ce qu'**un mélange approximativement équimolaire des composés A2 et B1, les radicaux R3 et R5 dans B1 désignant de préférence tous les deux un radical stéarate, est choisi, connu sous le nom de « distéarate de polyglycéryl(3)-méthylglucose ».

3. Préparations contenant des combinaisons d'agents actifs selon la revendication 1, ayant une teneur de 0,005 à 50,0 % en poids, notamment de 0,01 à 20,0 % en poids, de préférence de 0,02 à 10,0 % en poids, de manière particulièrement préférée de 0,02 à 5,0 % en poids, de manière tout particulièrement préférée de 0,1 à 3,0 % en poids, à chaque fois par rapport au poids total de la composition.

4. Utilisation selon la revendication 2, selon laquelle les combinaisons d'agents actifs sont présentes dans des préparations cosmétiques ou dermatologiques, en une teneur de 0,005 à 50,0 % en poids, notamment de 0,01 à 20,0 % en poids, de préférence de 0,02 à 10,0 % en poids, de manière particulièrement préférée de 0,02 à 5,0 % en poids, de manière tout particulièrement préférée de 0,1 à 3,0 % en poids, à chaque fois par rapport au poids total de la composition.

5. Préparations selon l'une quelconque des revendications précédentes, dans lesquelles un extrait de l'écorce de Magnolia officinalis est choisi en tant qu'extrait d'écorce de magnolia.

6. Utilisation selon l'une quelconque des revendications précédentes, dans laquelle un extrait de l'écorce de Magnolia officinalis est choisi en tant qu'extrait d'écorce de magnolia.
